# EUROPÄISCHE PATENTANMELDUNG

(11) **EP 3 974 003 A1**
(43) Veröffentlichungstag der Anmeldung: **30.03.2022**
(21) Anmeldenummer: 21190366.1
(22) Anmeldetag: 09.08.2021
(51) Int. Cl.: A61L 9/18

(54) **VORRICHTUNG ZUR DESINFEKTION VON LUFT MIT ELEKTROMAGNETISCHER STRAHLUNG**

(30) Priorität: 23.09.2020 DE 102020124739
(71) Anmelder: ebm-papst Mulfingen GmbH & Co. KG, 74673 Mulfingen (DE)
(72) Erfinder: Wystup, Ralph, 74653 Künzelsau (DE); Humm, Markus, 74679 Weißbach (DE); Nase, Rainer, 97990 Weikersheim (DE); Wystup, Frederik, 74632 Neuenstein (DE)
(74) Vertreter: Staeger & Sperling Partnerschaftsgesellschaft mbB

(57) **Zusammenfassung**

Die Erfindung betrifft eine Vorrichtung (1) zur Desinfektion von Luft mit elektromagnetischer Strahlung, aufweisend eine von Luft durchströmbare Strahlungskammer (10), welche von einer Ansaugseite (A) zu einer Ausblasseite (B) entlang eines Strömungspfades von Luft durchströmbar ist, und zumindest eine Strahlungsquelle (11), welche ausgebildet ist, eine elektromagnetische Strahlung im Mikrowellenbereich zu erzeugen und die elektromagnetische Strahlung in die Strahlungskammer (10) zu emittieren, so dass die durch die Strahlungskammer (10) strömende Luft mit der elektromagnetischen Strahlung beaufschlagt wird, und wobei die Vorrichtung (1) ferner zumindest einen Ventilator (20) mit einem Schaufelrad (21) zur Erzeugung einer Luftströmung durch die Strahlungskammer (10) aufweist, welcher ausgebildet ist, Luft an der Ansaugseite (A) anzusaugen, durch die Strahlungskammer (10) entlang des Strömungspfades zu der Ausblasseite (B) zu fördern und an der Ausblasseite (B) aus der Strahlungskammer (10) auszublasen, wobei zumindest das Schaufelrad (21) in der Strahlungskammer (10) angeordnet ist und das Schaufelrad (21) eine Vielzahl von Schaufeln (22) aufweist, welche zumindest abschnittsweise aus einem die elektromagnetische Strahlung ablenkendem Material gebildet sind.

## Beschreibung

Die Erfindung betrifft eine Vorrichtung zur Desinfektion von Luft mit elektromagnetischer Strahlung im Mikrowellenbereich.

Manche Bakterien und Viren, wie beispielsweise auch Covid-19, können als Aerosol oder an Wassertröpfchen anhaftend bzw. von diesen umhüllt in Luft vorhanden sein. Dementsprechend ist es wünschenswert, möglichst große Mengen von Luft insbesondere bei der Verwendung von Klimaanlagen oder generell in geschlossenen Räumen von diesen Bakterien und Viren reinigen zu können.

Im Stand der Technik sind bereits verschiedene Ansätze zur Desinfektion von Luft bekannt, welche jedoch meist nicht geeignet sind, große Mengen von Luft stetig zu desinfizieren, also die in der Luft vorhandenen Bakterien oder Viren unschädlich zu machen.

Beispielsweise ist bereits bekannt, die Luft mittels UVC-Licht zu desinfizieren. Zudem ist auch grundsätzlich die Desinfektion von Luft mittels einer Mikrowellenstrahlung oder Hitze bekannt. Die dafür im Stand der Technik vorgesehenen Vorrichtungen erlauben jedoch meist nur die Reinigung vergleichsweise geringer Luftmengen.

Der Erfindung liegt deshalb die Aufgabe zugrunde, die vorgenannten Nachteile zu überwinden und eine Vorrichtung sowie ein zugehöriges Verfahren bereitzustellen, durch welche möglichst große Mengen von Luft effektiv und effizient desinfiziert bzw. gereinigt werden können.

Diese Aufgabe wird durch die Merkmalskombination gemäß Patentanspruch 1 gelöst.

Erfindungsgemäß wird eine Vorrichtung zur Desinfektion von Luft mit elektromagnetischer Strahlung vorgeschlagen. Die Vorrichtung weist eine von Luft durchströmbare Strahlungskammer auf, welche von einer Ansaugseite zu einer Ausblasseite entlang eines Strömungspfades von Luft durchströmbar ist. Ferner weist die Vorrichtung zumindest eine Strahlungsquelle auf, welche ausgebildet ist, eine elektromagnetische Strahlung im Mikrowellenbereich zu erzeugen und die elektromagnetische Strahlung in die Strahlungskammer zu emittieren, wobei eine Frequenz der elektromagnetischen Strahlung derart gewählt ist, dass in der Luft vorhandene Wassermoleküle zu einer die Wassermoleküle erhitzenden Schwingung angeregt werden, so dass die durch die Strahlungskammer strömende Luft mit der elektromagnetischen Strahlung beaufschlagt und in der Luft vorhandenes Wasser auf eine Temperatur von zumindest 100°C gebracht bzw. erhitzt wird. Durch die Beaufschlagung der Luft mit der elektromagnetischen Strahlung bzw. mit der Mikrowellenstrahlung, wird die die Strahlungskammer durchströmende Luft desinfiziert. Wie einleitend beschrieben, sind die in der Luft vorhandenen Viren und Bakterien, welche im Weiteren auch als Partikel bezeichnet werden, meist als Aerosol oder in Wassertröpfchen anhaftend in Luft vorhanden. Somit ist immer Wasser bzw. sind immer Wassermoleküle vorhanden. Die meisten Viren und auch die meisten Bakterien können in kochendem Wasser abgetötet werden. Es muss also nur das Wasser in der Luft soweit erhitzt werden, dass die Partikel dabei abgetötet bzw. unschädlich gemacht werden. Die hier vorgeschlagene Vorrichtung bzw. das zugehörige Verfahren sehen vor, das Wasser in der Luft mittels elektromagentischer Strahlung im Mikrowellenbereich, also mit Mikrowellenstrahlung zu erhitzen.

Mikrowellen bzw. deren elektrische Feldkomponente erhitzen Materialen mit einem Dipolmoment, wie Wassermoleküle, indem sie die Moleküle in Drehschwingung versetzen, wobei hierbei die Frequenz der Mikrowollen bzw. der Strahlung nur insofern ausschlaggebend ist, als das sie nicht zu hoch sein darf, so dass die Moleküle der Drehbewegung des Wechselfeldes noch folgen können.

Die infolge des Skineffektes berechnete Eindringtiefe liegt in Wasser im cm Bereich (1cm - 5cm), je nach Temperatur des Wassers. Mit steigender Frequenz der Wellen bzw. der Strahlung nimmt die Eindringtiefe ab, aber die Energieumsetzung nimmt infolge der geringeren Wellenlänge zu. Die Eindringtiefe ist dabei definiert als die Tiefe in der die Anfangsintensität der Wellen auf 37% zurückgegangen ist. Das bedeutet, dass die Wellen auch in die Bereiche unterhalb der Eindringtiefe gelangen, aber die Erwärmung dort deutlich geringer ist als oberhalb. Optimal wäre also eine Frequenz, deren dazugehörige Eindringtiefe dem mittleren Radius der Partikelverteilung in der Luft um 5 µm bis 0,5 mm entspricht. Dies ist aber aufgrund der dazu sehr hohen Frequenz von über 30 GHz nicht wirtschaftlich möglich, da es hierfür kaum geeignete Strahlungsquellen gibt. Ebenso würden bei dieser hohen Frequenz die Wassermoleküle, wie beschrieben, keine ausreichende Drehbewegung mehr durchführen.

Daher ist zur Erhitzung von Wasser eine von einer Resonanzfrequenz abweichende und somit nicht optimale Absorptionsfrequenz von ca. 2,45 GHz ausreichend. Dabei muss die Frequenz der elektromagnetischen Strahlung nicht genau 2,45 GHz betragen, es ist jedoch vorteilhaft, wenn die Frequenz in einem Bereich um 2,45 GHz und beispielsweise zwischen 2,00 und 3,00 GHz ist. Als Strahlungsquelle zur Erzeugung einer solchen elektromagnetischen Strahlung im Bereich 2,45 GHz und vorzugsweise genau 2,45 GHz können Magnetrone verwendet werden, wie sie auch bei handelsüblichen Mikrowellenherden zum Einsatz kommen, so dass deren Bereitstellung äußerst wirtschaftlich möglich ist.

Die Verwendung von Mikrowellen bzw. elektromagnetischer Strahlung anstelle anderer im Stand der Technik bekannten Varianten, wie beispielsweies eine Desinfektion von Luft mittels UVC-Licht ist sinnvoll, da ein hoher Durchsatz von Luft erzielt werden kann.

Die Mikrowellenstrahlung selbst kann, anders als bei UVC-Licht, aufgrund ihrer geringen Energie organische Verbindungen nicht aufbrechen, dies ist bei Mikrowellen rein durch das Erhitzen infolge der Dipolschwingungen möglich. Die Erhitzung erfolgt also genauso, wie wenn man die Verbindung auf dem Herd erwärmen würde.

Dazu muss genügend Energie in kurzer Zeit in die Partikel eingebracht werden, da sich diese vorzugsweise nur kurz in der Strahlungskammer aufhalten bzw. aufhalten sollen.

Da handelsübliche Magnetrone bei ca. 60% Wirkungsgrad gut 500 Watt HF-Leistung einbringen können und mehrere Magnetrone bzw. Strahlungsquellen verwenden werden können, kann eine sehr hohe Strahlungsleistungen durch mehrere Strahlungsquellen in der Strahlungskammer erzielt werden.

Zur Steigerung der Effizienz ist vorgesehen, dass die Vorrichtung erfindungsgemäß ferner zumindest einen Ventilator mit einem Schaufelrad zur Erzeugung einer Luftströmung durch die Strahlungskammer aufweist. Der Ventilator ist ausgebildet, Luft bzw. genauer Umgebungsluft an der Ansaugseite anzusaugen, durch die Strahlungskammer entlang des Strömungspfades zu der Ausblasseite zu fördern und an der Ausblasseite aus der Strahlungskammer auszublasen. Dabei ist zumindest das Schaufelrad in der Strahlungskammer angeordnet, wobei das Schaufelrad eine Vielzahl von Schaufeln aufweist, welche zumindest abschnittsweise aus einem die elektromagnetische Strahlung ablenkendem bzw. reflektierenden Material gebildet sind. Die Schaufeln bzw. das Schaufelrad des Ventilators wirkt dadurch als ein von handelsüblichen Mikrowellen bekannter Reflektor, an welchem die von der zumindest einen Strahlungsquelle emittierten Strahlen reflektiert werden. Durch die Reflektion werden stehende Wellen innerhalb der Strahlungskammer vermieden, durch welche es sonst zu statischen heißen und kalten Zonen in der Strahlungskammer kommen würde. Zusätzlich zu der bewirkten Luftförderung entlang des Strömungspfades ist der Ventilator bzw. das Schaufelrad somit ein der Feldhomogenisierung dienender Reflektor.

Der Ventilator kann vollständig in der Strahlungskammer angeordnet sein, wobei hierbei ein das Schaufelrad rotatorisch antreibender Motor vorzugsweise gegen die elektromagnetische Strahlung in der Strahlungskammer abgeschirmt ist. Alternativ kann der Motor auch außerhalb der Strahlungskammer angeordnet sein und das Schaufelrad in der Strahlungskammer mittels einer von außen in die Strahlungskammer führender Antriebswelle antreiben. Ist sowohl ansaug- als auch ausblasseitig jeweils ein Ventilator oder zumindest jeweils ein Schaufelrad vorgesehen, können die Schaufelräder auch über eine gemeinsame Antriebswelle verbunden und angetrieben werden.

Um zu gewährleisten, dass eine ausreichend große Menge Luft bzw. ein ausreichend großer Volumenstrom desinfiziert werden kann, ist eine durch den Ventilator erzeugte Luftströmung durch die Strahlungskammer, die von Luft durchströmte Länge der Strahlungskammer und die Anzahl der Strahlungsquellen derart aufeinander angepasst, so dass die in der gewählten Luftmenge bzw. die in dem Volumenstrom enthaltenen Partikel mit einer ausreichend großen Strahlungsenergie beaufschlagt werden können und die gewünschte Menge Luft innerhalb einer vorbestimmten Zeit die Strahlungskammer durchströmen und dabei im Wesentlichen vollständig desinfiziert werden kann.

Vorzugsweise ist vorgesehen, dass die Strahlungskammer aus einem die elektromagnetische Strahlung dämpfenden bzw.abschirmenden und/oder reflektierenden Material gebildet ist, so dass keine elektromagnetische Strahlung aus der Strahlungskammer austreten kann.

Um zu vermeiden, dass Mikrowellenstrahlung an der Ansaug- oder der Ausblasseite aus der Strahlungskammer austritt, ist an der Ansaugseite und der Ausblasseite der Strahlungskammer jeweils ein von Luft durchströmbares Abschirmelement angeordnet ist, welches aus einem die elektromagnetische Strahlung dämpfenden bzw. abschirmenden und/oder reflektierenden Material gebildet ist. Vorzugsweise ist die Ansaug- und die Ausblasseite mit einem Metallgitter verschlossen, welches bzw. dessen Maschenweite entsprechend der Wellenlänge bzw. Frequenz der elektromagnetischen Strahlen gewählt ist.

Eine Variante kann zudem vorgesehen sein, dass das zumindest eine Schaufelrad integral das Abschirmelement auf der Ansaugseite bildet oder als ein zusätzliches Abschirmelement auf der Ansaugseite wirkt. Alternativ oder zusätzlich kann auch vorgesehen sein, dass das zumindest eine Schaufelrad oder ein weiteres Schaufelrad integral das Abschirmelement auf der Ausblasseite bildet.

Um die Verweilzeit der Luft bzw. die in der Luft vorhandenen Partikel in der Strahlungskammer und somit die Dauer der Bestrahlung zu erhöhen, ist das an der Ausblasseite der Strahlungskammer angeordnete Abschirmelement gemäß einer weiteren vorteilhaften Variante als Strömungshindernis ausgebildet, durch welches der Luftdruck in der Strahlungskammer erhöht wird. Hierfür kann durch das Abschirmelement beispielsweise einfach der Strömungsquerschnitt bzw. die von Luft durchströmbare Fläche reduziert sein. Die Verwendung eines Abschirmelements als Strömungshindernis führt zudem zu einer Druckerhöhung in der Strahlungskammer und folglich zugleich zu einer höheren Partikeldichte in dieser, was die Effizienz der Desinfektion steigert.

Die Strahlungskammer weist zudem bei einer ebenfalls vorteilhaften Weiterbildung zumindest abschnittsweise einen runden von Luft durchströmbaren Strömungsquerschnitt auf, wobei das zumindest eine Schaufelrad einen zu dem Strömungsquerschnitt korrespondierenden Durchmesser aufweist. Hierbei ist als korrespondierend zu verstehen, dass das Schaufelrad nach radial außen unmittelbar an eine den Strömungsquerschnitt definierende Wandung der Strahlungskammer angrenzt, der Durchmesser des runden Strömungsquerschnitts also nur unwesentlich größer ist, als der Außendurchmesser des Schaufelrades, wobei die Rotation des Schaufelrades ungehindert möglich ist.

Um sicherzustellen, dass alle Viren und/oder Bakterien bzw. alle Partikel der ansaugseitig in die Strahlungskammer gesaugten Luft in Wasser gehüllt sind oder zumindest an Wasser anhaften, sieht eine weitere Variante vor, dass die Vorrichtung insbesondere bei ansaugseitig sehr trockener Luft ferner zumindest eine Durch- bzw. Befeuchtungsvorrichtung aufweist, welche entlang dem Strömungspfad vor der Strahlungskammer oder ansaugseitig zumindest abschnittsweise in der Strahlungskammer angeordnet ist. Die Durchfeuchtungsvorrichtung ist ausgebildet, die die Strahlungskammer durchströmende Luft vor der Beaufschlagung mit den elektromagnetischen Strahlen zu befeuchten.

Beispielsweise kann die zumindest eine Durchfeuchtungsvorrichtung ein Ultraschall-Wasserzerstäuber sein.

Die Verwendung einer Durchfeuchtungsvorrichtung ist zudem in Kombination mit einem ansaugseitig der Strahlungskammer bzw. ansaugseitig in der Strahlungskammer angeordneten Ventilator vorteilhaft, da die befeuchtete Luft durch den Ventilator nochmals verwirbelt und somit durchmischt wird, bevor die Luft mit Strahlung beaufschlagt wird.

Insbesondere bei der Verwendung einer Durchfeuchtungsvorrichtung aber generell auch, wenn ausblasseitig möglichst trockene Luft oder Luft mit einer vorbestimmten Luftfeuchte ausgestoßen werden soll, ist eine weitere Ausführungsform der Vorrichtung von Vorteil, bei welcher diese ferner zumindest eine Entfeuchtungsvorrichtung aufweist, welche entlang dem Strömungspfad nach der Strahlungskammer oder ausblasseitig zumindest abschnittsweise in der Strahlungskammer angeordnet ist. Die Entfeuchtungsvorrichtung ist hierbei ausgebildet, die die Strahlungskammer durchströmende Luft nach der Beaufschlagung mit den elektromagnetischen Strahlen, also nach der Dekontamination zu entfeuchten, also der feuchten Luft Wasser zu entziehen.

Die zumindest eine Entfeuchtungsvorrichtung kann beispielsweise zumindest ein vorzugsweise plattenförmiger Kondenser sein, welcher auch als thermischer Kondensator bezeichnet werden kann. Ein solcher kann auch als eine oder mehrere Metallplatten ausgeführt sein, wobei der Kondenser durch Kondensation der Luft an den Platten der Luft Wasser entzieht.

In Verbindung mit einem Kondenser ist ausblasseitig zudem vorzugsweise ein Wasserauslas vorgesehen, durch welchen das am Kondenser aus der Luft abgeschiedene Wasser als Kondensat aus der Vorrichtung abgeführt werden kann.

Insbesondere, wenn die zumindest eine Strahlungsquelle und weiter vorzugsweise auch die zumindest eine Durchfeuchtungsvorrichtung zumindest mit einem Kühlkörper an einer außenseitigen Wandung der Strahlungskammer bzw. außenseitig an der Strahlungskammer angeordnet sind, ist eine weitere Ausführungsform von Vorteil, bei welcher die Vorrichtung einen entlang des Strömungspfades ansaugseitig an der Strahlungskammer angeordneten Luftzuführungskanal aufweist, welcher zumindest abschnittsweise die Strahlungskammer umschließt, so dass zumindest ein Teil der angesaugten Luft außenseitig an der Strahlungskammer entlang strömt und diese vorzugsweise kühlt. Sind die zumindest eine Strahlungsquelle und/oder die zumindest eine Durchfeuchtungsvorrichtung zumindest mit ihrem Kühlkörper außenseitig an der Strahlungskammer vorgesehen, liegen diese vorteilhaft innerhalb des Luftzuführungskanal, so dass angesaugte Luft an dem Kühlkörper oder den Kühlkörpern entlang Wärme aufnimmt, vorgeheizt wird und zugleich die Komponenten kühlt, bevor die Luft ansaugseitig in die Strahlungskammer gesaugt wird.

Wird die Vorrichtung mit anderen Anlagen, wie beispielsweise einer Klimaanlage kombiniert, kann auch vorgesehen sein, dass der ansaugseitig in die Strahlungskammer gesaugte Luftstrom ein Kühlluftstrom ist, welcher entlang des Strömungspfades vor der Strahlungskammer zur Kühlung von Bauteilen der anderen Anlage verwendet wird.

Ansaugseitig der Strahlungskammer kann zudem zumindest ein Vorfilter entlang bzw. in dem Strömungspfad vorgesehen sein, durch welchen Fremdkörper aus der Luft gefiltert werden können. Als Fremdkörper werden hierbei beispielsweise Staub- oder andere Fremdkörper verstanden, welche größer sind als die Partikel, welche durch die Bestrahlung in der Strahlungskammer unschädlich gemacht werden sollen und daher in der Strahlungskammer störend wirken würden.

Ein weiterer Aspekt der Erfindung betrifft zudem ein Verfahren zur Luftdesinfektion durch Mikrowellen mit einer erfindungsgemäßen Vorrichtung, wobei Luft ansaugseitig durch den Ventilator angesaugt und entlang dem Strömungspfad durch die Strahlungskammer zu der Ausblasseite gefördert und entlang des Strömungspfades in der Strahlungskammer mit der durch die zumindest eine Strahlungsquelle erzeugte elektromagnetischen Strahlung beaufschlagt wird, so dass in der Luft enthaltenes Wasser auf eine Temperatur von zumindest 100°C gebracht wird und von dem Wasser umgebene oder an diesem anhaftende Bakterien oder Viren unschädlich gemacht werden.

Die vorstehend offenbarten Merkmale sind beliebig kombinierbar, soweit dies technisch möglich ist und diese nicht im Widerspruch zueinander stehen.

Andere vorteilhafte Weiterbildungen der Erfindung sind in den Unteransprüchen gekennzeichnet bzw. werden nachstehend zusammen mit der Beschreibung der bevorzugten Ausführung der Erfindung anhand der Figur näher dargestellt. Es zeigt:

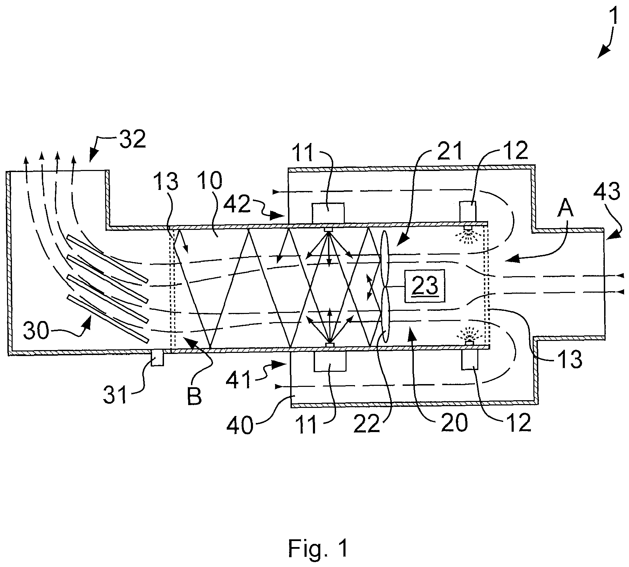

- Fig. 1: eine Vorrichtung zur Desinfektion von Luft mit elektromagnetischer Strahlung in einer Schnittdarstellung.

Die Figur ist beispielhaft schematisch und zeigte eine Vorrichtung 1 zur Desinfektion von Luft, welche im Wesentlichen aus drei Abschnitten bzw. Baugruppen gebildet ist. Die Strahlungskammer 10 bildet den zentralen Abschnitt bzw. eine erste Baugruppe. In die Strahlungskammer 10 wird durch einen strömungstechnisch vorgeschalteten Luftzuführungskanal 40 als eine zweite Baugruppe Außen- bzw. Umgebungsluft gesaugt. Nach einer Beaufschlagung der Luft mit elektromagnetischer Strahlung bzw. genauer mit Mikrowellen in der Strahlungskammer 10 zur Desinfektion der Luft, wird diese in eine strömungstechnische nachgeschaltete dritte Baugruppe geleitet bzw. geblasen, welche vorliegend zur Luftentfeuchtung mittels eines Entfeuchtungsvorrichtung 30 dient.

In bzw. angrenzend an die Strahlungskammer 10 sind vorliegend zwei Magnetrone als Strahlungsquellen 11 vorgesehen, welche jeweils elektromagnetische Strahlung im Mikrowellenbereich mit einer Frequenz von 2,45 GHz in die Strahlungskammer 10 emittieren, welche in der Strahlungskammer 10 durch die Pfeile angedeutet ist, welche durch die Außenwände der Strahlungskammer 10, durch die Abschirmelemente 13 und durch das Schaufelrad 21 bzw. dessen Schaufeln 22 reflektiert und die Umgebung somit von der elektromagnetischen Strahlung abgeschirmt wird.

Die Leistung der Strahlungsquellen und die Strömung der Luft durch die Strahlungskammer von deren Ansaugseite A zu deren Ausblasseite B ist derart aufeinander abgestimmt, dass das in der Luft enthaltene Wasser im wesentlichen vollständig zum Kochen gebracht wird, wodurch enthaltene Partikel, also Viren und Bakterien, im wesentlichen vollständig neutralisiert werden. Hierfür kann die Länge des Strömungsweges, die Geschwindigkeit der Strömung der Luft durch die Strahlungskammer 10, der Luftdruck in der Strahlungskammer 10 bzw. der Differenzdruck in der Strahlungskammer 10 gegenüber einer Umgebung der Vorrichtung 1 sowie beispielsweise auch die Luftfeuchtigkeit der ansaugseitig in die Strahlungskammer 10 strömenden Luft berücksichtigt werden.

Um besonders bei ansaugseitig trockener Luft ausreichend Wasser bzw. eine ausreichende Luftfeuchte der bestrahlten Luft vorauszusetzen, sind bei der dargestellten Variante zwei Durchfeuchtungsvorrichtungen 12 in Form von Ultraschall-Wasserzerstäubern vorgesehen, welche die auf ein Ansaugseite A in die Strahlungskammer 10 einströmende Luft befeuchten. Hierbei ist insbesondere auch vorteilhaft, dass in Strömungsrichtung nach den Durchfeuchtungsvorrichtungen 12 der Ventilator 20 vorgesehen ist, welcher die befeuchtete Luft verwirbelt und somit die Durchmischung der Luft mit Feuchtigkeit erhöht.

Vorteilhaft ist zudem, dass die Schaufeln 22 des Schaufelrades 21 des Ventilators 20 zumindest abschnittsweise aus einem die elektromagnetische Strahlung reflektierendem Material, wie beispielsweise Metall, gebildet sind, so dass das Schaufelrad 21 integral als rotierender Reflektor dient, durch welchen die elektromagnetischen Strahlen in der Strahlungskammer 10 chaotisch ausgelenkt werden. Durch die chaotische Reflektion werden innerhalb der Strahlungskammer 10 stehende Wellen vermieden, so dass es nicht zu heißen und kalten Zonen kommt und die durchströmende Luft gleichmäßig erhitzt bzw. das darin enthaltene Wasser vollständig zum Kochen gebracht wird.

Der Ventilator 20 weist zum Antrieb seines Schaufelrades 21 einen Motor 23 auf, welcher bei der abgebildeten Ausführungsform ebenfalls in der Strahlungskammer 10 angeordnet ist, aber alternativ auch außerhalb angeordnet sein kann. Obgleich der Motor 23 vorzugsweise auf einer von den Strahlungsquellen 11 abgewandten Seite des Schaufelrades 21 und dadurch vor der elektromagnetischen Strahlung abgeschirmt angeordnet ist, da das Schaufelrad 21 als ein Abschirmelement wirkt, kann der Motor 23 zusätzlich eine eigene Schirmung aufweisen und vor elektromagnetischen Strahlen abgeschirmt sein.

Abhängig davon, wie umfassend der Abschirmeffekt durch das Schaufelrad 21 ist, kann auf das auf der Ansaugseite A angeordnete Abschirmelement 13 verzichtet werden, da seine Funktion dann integral durch das Schaufelrad 21 übernommen wird.

Vorliegend sind zwei Magnetrone als Strahlungsquellen 11 vorgesehen, welche einander gegenüberliegend dargestellt sind. Abhängig von der einzubringenden Strahlungsleistung und auch abhängig von dem zu desinfizierenden Volumenstrom, können insbesondere bei einer Strahlungskammer 10 mit einem runden Querschnitt in Umfangsrichtung eine Vielzahl von vorzugsweise gleichmäßig verteilten Strahlungsquellen 11 vorgesehen sein. Zusätzlich können diese auch entlang des Strömungspfades zueinander benachbart angeordnet sein und sich über die Länge der Strahlungskammer 10 verteilen.

Bei der in Figur 1 dargestellten Variante der Vorrichtung 1 sind die Strahlungsquellen 11 sowie die Durchfeuchtungsvorrichtungen 12 bzw. zumindest Kühlelemente dieser Komponenten außenseitig an der Außenwandung der Strahlungskammer 10 angeordnet. Der Luftzuführungskanal 40 umgibt in dem Abschnitt, in welchem diese Komponenten angeordnet sind, die Strahlungskammer 10, so dass zumindest ein Teil der Luftströmung, welche durch den Ventilator 20 aus der Umgebung der Vorrichtung 1 in die Strahlungskammer 10 gesaugt wird, kühlend an diesen Komponenten entlang führt.

Die Luftströmung ist in der Abbildung als gestrichelte Pfeile dargestellt, wobei die Luftströme, welche durch die Einströmöffnungen 41, 42 angesaugt werden, die Strahlungsquellen 11 und die Durchfeuchtungsvorrichtungen 12 kühlen und dadurch zugleich vorgeheizt werden, so dass die in die Strahlungskammer 10 einströmende Luft bzw. das in der einströmenden Luft enthaltene Wasser in der Strahlungskammer 10 schneller zum Kochen gebracht werden kann. Obgleich die einströmende Luft auch vollständig zur Kühlung außerhalb der Strahlungskammer 10 angeordneter Komponenten genutzt werden kann, ist vorliegend vorgesehen, dass zusätzlich Luft durch eine weitere Einströmöffnung 43 eingesaugt wird.

Im Übrigen kann der Luftzuführungskanal 40 die Strahlungskammer 10 auch vollständig ringförmig umlaufen, so dass die beiden Eintströmöffnungen 41, 42 einen ringförmigen Einlass bzw. eine einzige ringförmige Einströmöffnung bilden.

Die gestrichelt dargestellte die Vorrichtung 1 durchströmende Luft wird auf der Ausblasseite B aus der Strahlungskammer 10 in eine Entfeuchtungsvorrichtung 30 geblasen, welche vorliegend als vier benachbart zueinander angeordnete thermische Kondensatoren ausgebildet ist. Das in der Luft vorhandene Wasser bzw. die feuchte, Wasserdampf belastete Luft wird durch diese Entfeuchtungsvorrichtung 30 entfeuchtet, so dass an dem Auslass 32 der Vorrichtung 1 die Luft mit einer vorbestimmten Luftfeuchtigkeit ausströmen kann. Dass an den Kondensatoren kondensierende Wasser kann aus der Vorrichtung 1 über eine Ablauföffnung 31 abgeführt werden.

Die Erfindung beschränkt sich in ihrer Ausführung nicht auf die vorstehend angegebenen bevorzugten Ausführungsbeispiele. Vielmehr ist eine Anzahl von Varianten denkbar, welche von der dargestellten Lösung auch bei grundsätzlich anders gearteten Ausführungen Gebrauch macht.

## Patentansprüche

1. Vorrichtung (1) zur Desinfektion von Luft mit elektromagnetischer Strahlung,
aufweisend eine von Luft durchströmbare Strahlungskammer (10), welche von einer Ansaugseite (A) zu einer Ausblasseite (B) entlang eines Strömungspfades von Luft durchströmbar ist, und
zumindest eine Strahlungsquelle (11), welche ausgebildet ist, eine elektromagnetische Strahlung im Mikrowellenbereich zu erzeugen dass und die elektromagnetische Strahlung in die Strahlungskammer (10) zu emittieren, wobei eine Frequenz der elektromagnetischen Strahlung derart gewählt ist, dass in der Luft vorhandene Wassermoleküle zu einer die Wassermoleküle erhitzenden Schwingung angeregt werden, so dass die durch die Strahlungskammer (10) strömende Luft mit der elektromagnetischen Strahlung beaufschlagt und in der Luft vorhandenes Wasser auf eine Temperatur von zumindest 100°C gebracht wird,
und wobei die Vorrichtung (1) ferner zumindest einen Ventilator (20) mit einem Schaufelrad (21) zur Erzeugung einer Luftströmung durch die Strahlungskammer (10) aufweist, welcher ausgebildet ist, Luft an der Ansaugseite (A) anzusaugen, durch die Strahlungskammer (10) entlang des Strömungspfades zu der Ausblasseite (B) zu fördern und an der Ausblasseite (B) aus der Strahlungskammer (10) auszublasen,
wobei zumindest das Schaufelrad (21) in der Strahlungskammer (10) angeordnet ist und das Schaufelrad (21) eine Vielzahl von Schaufeln (22) aufweist, welche zumindest abschnittsweise aus einem die elektromagnetische Strahlung ablenkendem Material gebildet sind.

2. Vorrichtung nach Anspruch 1,
wobei an der Ansaugseite (A) und der Ausblasseite (B) der Strahlungskammer (10) jeweils ein von Luft durchströmbares Abschirmelement (13) angeordnet ist, welches aus einem die elektromagnetische Strahlung dämpfenden und/oder reflektierenden Material gebildet ist.

3. Vorrichtung nach Anspruch 2,
wobei das zumindest eine Schaufelrad (21) integral das Abschirmelement (13) auf der Ansaugseite (A) bildet.

4. Vorrichtung nach dem Anspruch 2 oder 3,
wobei das an der Ausblasseite (B) der Strahlungskammer (10) angeordnete Abschirmelement (13) als Strömungshindernis ausgebildet ist, durch welches der Luftdruck in der Strahlungskammer (10) erhöht wird.

5. Vorrichtung nach einem der vorhergehenden Ansprüche,
wobei die Strahlungskammer (10) einen runden von Luft durchströmbaren Strömungsquerschnitt aufweist und das zumindest eine Schaufelrad (21) einen zu dem Strömungsquerschnitt korrespondierenden Durchmesser aufweist.

6. Vorrichtung nach einem der vorhergehenden Ansprüche,
ferner aufweisend zumindest eine Durchfeuchtungsvorrichtung (12), welche entlang dem Strömungspfad vor der Strahlungskammer (10) oder ansaugseitig zumindest abschnittsweise in der Strahlungskammer (10) angeordnet ist und welche ausgebildet ist, die die Strahlungskammer (10) durchströmende Luft vor der Beaufschlagung mit den elektromagnetischen Strahlen zu befeuchten.

7. Vorrichtung nach dem vorhergehenden Anspruch,
wobei die zumindest eine Durchfeuchtungsvorrichtung (12) ein Ultraschall-Wasserzerstäuber ist.

8. Vorrichtung nach einem der vorhergehenden Ansprüche,
ferner aufweisend zumindest eine Entfeuchtungsvorrichtung (30), welche entlang dem Strömungspfad nach der Strahlungskammer (10) oder ausblasseitig zumindest abschnittsweise in der Strahlungskammer (10) angeordnet ist und welche ausgebildet ist, die die Strahlungskammer (10) durchströmende Luft nach der Beaufschlagung mit den elektromagnetischen Strahlen zu entfeuchten.

9. Vorrichtung nach dem vorhergehenden Anspruch,
wobei die zumindest eine Entfeuchtungsvorrichtung (30) ein Kondenser ist.

10. Vorrichtung nach einem der vorhergehenden Ansprüche,
ferner aufweisend einen entlang des Strömungspfades ansaugseitig an der Strahlungskammer (10) angeordneten Luftzuführungskanal (40), welcher zumindest abschnittsweise die Strahlungskammer (10) umschließt, so dass zumindest ein Teil der angesaugten Luft außenseitig an der Strahlungskammer (10) entlang strömt.

11. Verfahren zur Luftdesinfektion durch Mikrowellen mit einer Vorrichtung (1) gemäß einem der vorhergehenden Ansprüche,
wobei Luft ansaugseitig durch den Ventilator (20) angesaugt und entlang dem Strömungspfad durch die Strahlungskammer (10) zu der Ausblasseite (B) gefördert und entlang des Strömungspfades in der Strahlungskammer (10) mit der elektromagnetischen Strahlung beaufschlagt wird, so dass in der Luft enthaltenes Wasser auf eine Temperatur von zumindest 100°C gebracht wird.
